# EUROPEAN PATENT SPECIFICATION

(11) **EP 3 453 380 B1**
(45) Date of publication and mention of the grant of the patent: **03.12.2025**
(21) Application number: 17189777.0
(22) Date of filing: 07.09.2017
(51) Int. Cl.: A61K 8/44, A61K 8/46, A61Q 5/08, A61K 8/22, A61K 8/23

(54) **BLEACHING AND DECOLORIZATION PROCESS FOR KERATIN FIBERS**
BLEICH- UND ENTFÄRBUNGSVERFAHREN FÜR KERATINFASERN
PROCÉDÉ DE BLANCHIMENT ET DE DÉCOLORATION DE FIBRES KÉRATINIQUES

(43) Date of publication of application: 13.03.2019
(73) Proprietor: Kao Germany GmbH, 64297 Darmstadt (DE)
(72) Inventor: BAUER, Peter, 64297 Darmstadt (DE); Ghiasi, Fariba, 64297 Darmstadt (DE); BREAKSPEAR, Steven, 64297 DARMSTADT (DE); NÖCKER, Bernd, 64297 DARMSTADT (DE)

(56) References cited:
- EP-A2- 2 497 536
- WO-A2-2012/025269
- US-A1- 2017 112 740

## Description

The present invention is directed to a bleaching and/or decolorization process for keratin fibers, preferably human keratin fibers, more preferably human hair, which combines a reductive pre-treatment step followed by an application of oxidative compositions. Furthermore, the invention is directed to a kit-of-parts for bleaching and/or decolorizing keratin fibers.

The term bleaching within the meaning of the present invention is the process of lightening natural keratin fibers which have not undergone prior dyeing processes. The term decolorization describes the process of lightening artificially colored keratin fibers and, thus, is directed to the dimension of destroying remaining virgin keratin dyes and artificial dyes within the keratin fiber structure.

Many consumers are dissatisfied with their natural hair and wish to change the appearance of their hair to a more fashionable style. Especially for consumers with dark hair color, the hair color must be lightened prior to applying artificial hair color. During such routines, bleaching compositions are commonly applied either on the whole head or on individual hair streaks. However, common bleaching techniques reach a limitation in their lightening power without conferring a high degree of damage to the customer's hair. Another aspect is decolorization which points to the desire of customers to immediately lose their artificial hair color. Thus, there is an unfulfilled desire of the customer to receive bleaching and/or decolorization services with high lightening power while conferring a low degree of damage to the hair.

The inventors of the present invention have unexpectedly found that the combination of a pre-reduction step prior to applying bleaching compositions leads to strong lightening of keratin fibers. Furthermore, the combination of both steps leads to sufficient decolorization of artificially colored keratin fibers. It was further found that the inventive process did not lead to increased keratin fiber damage and reduced cosmetic feel of keratin fibers.

The combination of pre-reduction steps prior to bleaching of hair is known from WO2012/025269. The document discloses a pre-reduction step with an organic thiol compound in the time frame from 5 min to 45 min followed by a bleaching step. However, such a long reduction step has the disadvantage of conferring a high degree of damage to hair while not leading to a higher bleaching performance.

DE3433926 discloses a bleaching process for wool which combines a pre-reduction with an inorganic sulfur compound in a time frame of 15 min to 20 min at elevated temperature of up to 80°C. After the pre-treatment, an oxidative composition comprising hydrogen peroxide is added to the wool and the bleaching process is allowed to proceed for another 60 min at elevated temperature. The differences to the present invention are the processing times and temperature ranges.

US5264001 discloses a bleaching process for wool which allows for an oxidative treatment with hydrogen peroxide for 60 min at 60°C prior to adding an organic reducing compound. The reaction is allowed to proceed for another 25 min at elevated temperature in presence of the reducing agent. The differences to the present invention are the order of process steps and the processing times.

WO90/08216 discloses a bleaching process for wool which allows for an oxidative treatment with hydrogen peroxide in presence of a persulfate salt at 60°C for 60 min. After the oxidative treatment, an organic reducing agent is added and the treatment is allowed to proceed for 20 min at elevated temperature. The differences to the present invention are the order of process steps and the processing times.

In summary, none of the prior art documents solves the addressed problems in a satisfactory manner.

Therefore, the first object of the present invention is a bleaching and/or decolorization process for keratin fibers, preferably human keratin fibers, more preferably human hair, characterized in that it comprises the steps of:
a) applying to keratin fibers an aqueous composition comprising a reducing agent at a total concentration in the range of 0.1% to 2% by weight, calculated to the total of the composition, and leaving it onto hair for a time period in the range of 0.5 min to less than 5 min,
b) optionally rinsing-off the keratin fibers,
c) applying to keratin fibers an aqueous oxidizing composition resulting from mixing two compositions prior to application onto hair wherein one of the compositions is an anhydrous composition and comprises one or more peroxy salt(s) and/or persalt(s) and an alkalizing agent, and the other composition is an aqueous composition comprising an oxidizing agent, preferably hydrogen peroxide, wherein the mixture has a pH in the range of 8 to 12 and is left onto hair for a time period of 1 min to 45 min,
d) rinsing-off the keratin fibers,
e) optionally shampooing the keratin fibers,
f) optionally drying the keratin fibers.

The second object of the present invention is a kit-of-parts for bleaching and/or decolorizing keratin fibers, preferably human keratin fibers, more preferably human hair, characterized in that it comprises in a first separately packed container a composition as defined above for step a), in a second separately packed container an anhydrous composition as defined for step c), and in a third separately packed container a aqueous composition comprising an oxidizing agent, preferably hydrogen peroxide, as defined above for step c).

In another preferred embodiment of the inventive process disclosed above, the time period for step a) is in the range of 1 min to 4 min.

In a further preferred embodiment of the inventive process the keratin fibers are rinsed off after step a) and prior to applying the composition according to step c).

In another preferred embodiment of the inventive process the keratin fibers are not rinsed off after step a).

In principle, all reducing agents are suitable for the purpose of the aqueous composition of process step a) for the present invention.

The aqueous reducing composition of step a) comprises reducing agents selected from organic and/or inorganic reducing agents and/or their mixtures.

Preferred organic reducing agents are selected from thioglycolic acid and/or its salts, thiolactic acid and/or its salts, cysteamine and/or its salts, cysteine and/or its salts, thiourea and/or its salts, formamidine sulfinic acid and/or its salts.

Preferred inorganic reducing agents are selected from sulphite salts, bisulfite salts, dithionate salts.

The total concentration of reducing agents in the composition of step a) is in the range of 0.1% to 2% by weight, preferably 0.2% to 1.8% by weight, more preferably 0.5% to less than 1.5% by weight, calculated to the total of the composition of step a).

The pH of the composition of step a) is in the range of 2 to 11, preferably it is in the range of 7 to 10.5, more preferably in the range of 8 to 10.

Suitable acids for adjusting the pH of the composition of step a) are listed together with the ones for aqueous oxidizing composition of the step c) below.

For adjusting the pH of the composition of step a), the composition comprises one or more alkalizing agent(s), preferably selected from ammonia and/or its salts and/or aminomethyl propanol and/or an organic alkyl and/or alkanol amine and/or their salts according to the general structure 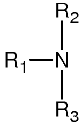 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H.

Suitable alkalizing agents are ammonia, monoethanolamine, diethanolamine, triethanolamine, monoethanol methylamine, monoethanoldimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, aminomethyl propanol and diethanolbutylamine. Preferred alkalizing agents are monoethanolamine and/or aminomethyl propanol. The most preferred is aminomethyl propanol abbreviated as AMP.

The reducing composition of step a) further comprises an organic solvent. In principle, all water-miscible organic solvents are suitable for the purpose of the invention. Suitable non-limiting examples are C2-C4 monoalcohols and/or C2-3 dialcohols and/or and glycerol. Preferred C2-C4 monoalcohols and/or C2-3 dialcohols solvents are ethanol, n-propanol, isopropanol, butanol, ethylene glycol, propylene glycol. Further suitable aromatic alcohols are phenoxyethanol and benzyl alcohol.

The total concentration of organic solvents in the composition of step a) is in the range of 0.5% to 10% by weight, preferably 1% to 8% by weight, more preferably 1.5% to 5% by weight, calculated to the total of the composition.

In principle, the reducing composition of step a) and/ or aqueous oxidizing composition of c) can be in any cosmetically acceptable form such as solution, thickened gel, dispersion, and/or emulsion.

It is preferred that the aqueous reducing composition of step a) and/or the aqueous oxidatizing composition of step c) are an emulsion and comprise one or more lipophilic compounds and a surfactant as an emulsifier.

Lipophilic compounds within the meaning of the present invention are compounds which do not fully mix with water at 25°C under atmospheric pressure.

For formation of emulsion, the composition comprises lipophilic compounds selected from natural and/or vegetable oils, petrolatum-based compounds, linear or branched, saturated or unsaturated fatty alcohols with C12 to C22, and fatty acid esters consisting of linear or branched, saturated or unsaturated fatty acids with C12 to C22 being esterified with linear or branched primary alcohols with C3 to C12, and silicones.

Suitable natural and/or vegetable oils are olive oil, almond oil, avocado oil, wheatgerm oil, and castor oil.

Suitable petrolatum-based compounds are liquid paraffins, especially paraffinum perliquidum and paraffinum subliquidum, and mineral oil, in particular white mineral oil.

Suitable fatty compounds are selected from linear or branched, saturated or unsaturated fatty alcohols with C12 to C22 are lauryl alcohol, tridecyl alcohol, myristyl alcohol, pentadecyl alcohol, cetyl alcohol, palmitoleyl alcohol, heptadecyl alcohol, stearyl alcohol, nonadecyl alcohol, arachidyl alcohol, behenyl alcohol, and/or their mixtures, such as cetearyl alcohol.

Suitable examples for fatty acid esters consisting of linear or branched, saturated or unsaturated fatty acids with C12 to C22 being esterified with linear or branched primary alcohols with C3 to C18 are octyl palmitate, isocetyl palmitate, isopropyl palmitate, octyl stearate, oleyl oleate, and myristyl myristate, as well as their mixtures.

The composition also comprises lipophilic ingredients such as silicones for example linear polysiloxanes such as dimethicones with various consistency and dimethiconols, aminated silicones with primary, secondary, tertiary or quaternary ammonium groups such as amodimethicone, polysilicone 9, and quaternium 80, cyclic silicones such as cyclomethicones, arylated silicones such as phenyl trimethicone; C10- to C36-fatty acid triglycerides, as well as their mixtures.

Total concentration of these lipophilic compounds is in the range of 1% to 20% by weight, preferably from 2% to 15% by weight, and more preferably from 3% to 10% by weight, calculated to the total of the compositions of step a) or c).

The compositions of step a) and/or c) comprise one or more surfactants. In principle, all surfactants may be added to the compositions such as cationic, anionic, non-ioninc, zwitterionic/amphoteric surfactants. The preferred surfactants are non-ionic and/or anionic surfactants.

Suitable non-ionic surfactants are in general all commonly known non-ionic surfactants available on the market.

Suitable nonionic surfactants are alkyl polyglycosides according to the general structure:

R²³O(R²⁴O)ₜZₓ

Wherein Z denotes a reducing carbohydrate with C₅ to C₆, R²³ is an alkyl group with C₈ to C₁₈, R²⁴ is ethyl or propyl, t ranges from 0 to 10, and x ranges from 1 to 5. Suitable compounds according to this structure are C₉-C₁₁ alkylpolyglycoside, the structures disclosed in EP-A 70 074, and JP 2015-123019A.

The preferred compound according to the structure of above is decyl glucoside.

Suitable examples for non-ionic surfactants are fatty alcohol ethoxylates of the following general structure

R¹⁸ (OCH₂CH₂)ₙ₄ OH

wherein R¹⁸ is straight or branched, saturated or unsaturated alkyl chain which may be synthetic or natural with a C chain length in the range of 8 to 40, preferably 9 to 30 and more preferably 9 to 24 and n4 is a number in the range of 5 to 40, preferably 9 to 30.

Further suitable nonionic surfactants are polypropylene glycol ethers of fatty alcohol according to general structure

R¹⁹ (OCH₂ (CH₃) CH₂)ₙ₅ OH

wherein R¹⁹ is straight or branched, saturated or unsaturated fatty alcohol which may be synthetic or natural with a C chain length in the range of 8 to 40, preferably 9 to 30 and more preferably 9 to 24 and n5 is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

R²⁰ C(O) (OCH₂CH₂)ₙ₆ OH

wherein R²⁰ is straight or branched, saturated or unsaturated alkyl group which may be synthetic or natural with a C chain length in the range of 7 to 39, preferably 9 to 29 and more preferably 9 to 23 and n6 is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate,

PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

R²¹ C(O) (OCH₂(CH₃) CH₂)ₙ₈ OH

wherein R²¹ is straight or branched, saturated or unsaturated alkyl group which may be synthetic or natural with a C chain length in the range of 7 to 39, preferably 9 to 29 and more preferably 9 to 23 and n8 is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are polyethylene glycol and polypropylene glycol ether of fatty alcohols of the following general structure

R²² (OCH₂ (CH₃) CH₂)ₙ₉ (OCH₂CH₂)ₙ₁₀ OH

wherein R²² is straight or branched, saturated or unsaturated alkyl group which may be synthetic or natural with a C chain length in the range of 7 to 39, preferably 9 to 29 and more preferably 9 to 23 and n9 and n10 may be the same or different and are a number in the range of 1 to 40.

Further suitable nonionic surfactants are ethoxylated triglycerides. Well known and commonly used examples are ethoxylated castor oil such as PEG-40 hydrogenated castor oil or and PEG-60 hydrogenated castor oil.

Suitable anionic surfactants are alkyl sulfate or preferably ethoxylated alkyl ether sulfate surfactant or mixtures thereof with an alkyl chain length of C₁₀ to C₂₂.

Suitable surfactants are laureth sulfates, coceth sulfate, pareth sulfate, capryleth sulphate, myreth sulfate, oleth sulfate, deceth sulfate, trideceth sulfate, coco sulphate, C₁₀-C₁₆ alkyl sulphate, C₁₁-C₁₅ alkyl sulphate, C₁₂-C₁₈ alkyl sulphate, C₁₂-C₁₅ alkyl sulphate, C₁₂-C₁₆ alkyl sulphate, C₁₂-C₁₃ alkyl sulfate, lauryl sulphate, myristyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Cations for the surfactants may be selected from sodium, potassium, magnesium and/or ammonium.

Suitable cationic surfactants are according to the general structure 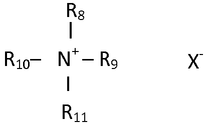 wherein R8 is a saturated or unsaturated, branched or linear alkyl chain with 8-22 C atoms or

R₁₂ CO NH (CH₂)n

where R₁₂ is saturated or unsaturated, branched or linear alkyl chain with 7-21 C atoms and n has typical value of 1 - 4 or

R₁₃ CO O (CH₂)n

where R₁₃ is saturated or unsaturated, branched or linear alkyl chain with 7-21 C atoms and n has typical value of 1 - 4, and
R₉ is H or unsaturated or saturated, branched or linear alkyl chain with 1 - 22 C atoms or

   R₁₂ CO NH (CH₂)n

   or

   R₁₃ CO O (CH₂)n
where R₁₂, R₁₃ and n are same as above.

R₁₀ and R₁₁ are H or lower alkyl chain with 1 to 4 Carbon atoms, and X is typically chloride, bromide, methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Suitable amphoteric and/or zwitterionic surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Total concentration of surfactants in the compositions of step a) and/or c) in in the range of 0.5% to 15% by weight, preferably 1% to 12% by weight, more preferably 1.5% to 10% by weight, calculated to the total of the compositions of step a) and/or c).

The viscosities of the composition of step a) and/or c) may be adjusted with a thickener. The preferred thickeners are selected from associative and/or non-associative thickeners.

In a further preferred embodiment of the present invention, the composition comprises one or more thickening polymers selected from anionic, nonionic, cationic and amphoteric polymers, preferably selected from polymers resulting in a solution and/or dispersion with a viscosity of at least 500 mPa•s measured at a polymer concentration of 1% by weight in water and at 20°C with a Brookfield viscometer, such as at 10 rpm for 1 min, with an appropriate spindle.

Suitable polymers are cellulose polymers, alginates, polysaccharides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

The preferred polymers are dehydroxanthan gum, xanthan gum, guar gum, and polymeric anionic thickeners, namely Carbomer and its derivatives.

Of particular advantageous use are thickeners which are commonly known as associative thickeners. Preferred are copolymers and/or crosspolymers which comprise an acrylate and/or methacrylate monomer unit and at least one more hydrophobic unit such as alkyl chains. Examples are acrylates/c10-30 alkyl acrylate crosspolymer, acrylates/steareth-20 methacrylate copolymer, acrylates/stearyl acrylate/dimethicone methacrylate copolymer, acrylates/beheneth-25 methacrylate copolymer, acrylates/lauryl acrylate/stearyl acrylate/ ethylamine oxide methacrylate copolymer.

The compositions of step a) and/or c) preferably comprise thickening agents at a total concentration in the range of 0.1% to 5%, preferably, 0.2% to 3%, more preferably 0.25% to 2.5% and most preferably 0.3% to 2% by weight calculated to the total of the compositions of step a) and/or c).

The oxidizing agent of the aqueous oxidizing composition of step c) is hydrogen peroxide and preferably at a concentration in the range of 1% to 20% by weight, more preferably in the range of 2% to 15% by weight, most preferably in the range of 3% to 12% by weight, calculated to the total of the aqueous oxidizing composition.

The pH of the aqueous oxidizing composition of step c) prior to mixing with the anhydrous composition is in the range of 1 to 6, more preferably 1.5 to 5, most preferably in the range of 1.8 to 4.

In principle, the pH of the compositions a) and c) can be adjusted with any organic and/or inorganic acid(s) or base or their mixtures. Suitable acids are phosphoric acid, hydrochloric acid as the inorganic ones and to the organic acids the well-known citric acid and lactic acid, glycolic acid, glyoxylic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Suitable bases are sodium hydroxide or potassium hydroxide. The most preferred acid for composition c) is phosphoric acid and for composition a) is citric acid.

The aqueous oxidizing composition of step c) is mixed with an anhydrous composition comprising one or more peroxy salt(s) and/or persalt(s) and an alkalizing agent prior to application onto keratin fibers.

The anhydrous composition may be in liquid or solid form. The term anhydrous means that the composition does not comprise any added water. It should be noted that one or more ingredients of the anhydrous composition may comprise bound crystal water or residual moisture which is not covered by the term.

This anhydrous composition comprises persalts and/or peroxy salts. Useful persalts and/or peroxy salts are sodium persulfate and potassium persulfate and ammonium persulfate, earth alkali peroxides such as magnesium peroxide, melamine peroxide or urea peroxide or phtholimidoperoxy hexanoic acid. The preferred persalts are sodium persulfate and/or potassium persulfate and/or ammonium persulfate. The total concentration of persalts and/or peroxysalts in the anhydrous composition is in the range of 10 to 80%, preferably 15 to 70%, more preferably 20 to 60% and most preferably 25 to 60% by weight, calculated to total of the anhydrous composition.

The anhydrous composition further comprises one or more alkalizing agents, which is preferably sodium metasilicate. The total concentration of alkalizing agents in the anhydrous composition is in the range from 1 to 20% by weight, calculated to the total of composition the anhydrous composition.

The anhydrous composition may comprise one or more ammonium salt(s) other than persalt(s) and peroxy salt(s). Suitable are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate. The anhydrous composition may also comprise mixtures of ammonium salts.

Preferred thereof are the ammonium phosphates, such as ammonium dihydrogen phopshate, diammonium hydrogen phosphate, diammonium sodium phosphate, ammonium sodium hydrogen phosphate or ammonium disodium phosphate, ammonium chloride, ammonium sulfate and diammonium hydrogen citrate.

The total concentration of one or more ammonium salt(s) other than persalt(s) and peroxy salt(s) in the anhydrous composition is in the range from 1 to 20% by weight, calculated to the total of composition the anhydrous composition.

The anhydrous composition and the aqueous oxidizing composition of step c) are mixed directly prior to use onto keratin fibers. In this case, the pH of the ready-to-use mixture is in the range of 8 to 12, preferably 8 to 11, more preferably 9 to 10.5.

The anhydrous composition may comprise one or more hair dyes selected from hair direct dyes. The hair direct dyes are selected from anionic, cationic and neutral dyes.

Suitable anionic direct dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, HC Blue 18, HC Red 18 and HC yellow 16 and their alkali metal salts such as sodium, potassium. Among those, the most preferred anionic dyestuffs are Acid Red 52, DC Violet 2, DC Red 33, DC Orange 4, DC Red 27, DC Yellow 10, HC Blue 18, HC Red 18 and HC Yellow 16.

Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87, Basic Orange 31 and HC Blue 17. The most preferred ones are Basic Red 51, Basic Yellow 87 and Basic Orange 31 sold by BASF, and HC Blue 17.

Suitable neutral nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

The anhydrous composition comprises one or more hair direct dye at a total concentration in the range of 0.01 to 10%, preferably 0.05 to 7.5% and more preferably 0.1 to 5% by weight calculated to the total of the anhydrous composition. The composition may also comprise mixtures of several direct dyes i.e. an anionic, a cationic and/or a nonionic ones. In such a case the dyes may be mixed at any ratio with each other.

The following examples are to illustrate the invention, but not to limit it.

### EXAMPLES

### Example 1

The following pre-treatment composition for step a) was prepared by conventional formulation techniques:

| | **% by weight** |
|---|---|
| Cetearyl alcohol | 10.0 |
| 2-amino-2-methylpropanol | 1.8 |
| Disodium hydrogenphophate | 0.5 |
| Thioglycolic acid | 1.5 |
| Acrylates copolymer | 0.5 |
| Sodium laureth sulfate | 1.0 |
| EDTA | 0.1 |
| Glycerol | 0.5 |
| Ammonia 25% | q.s. ad pH 9.4 |
| Water | ad 100.0 |

The following oxidative composition comprising hydrogen peroxide was prepared by conventional formulation techniques:

| | **% by weight** |
|---|---|
| Hydrogen peroxide | 9.0 |
| Phosphoric acid | q.s. ad pH 3.5 |
| Water | ad 100.0 |

The following anhydrous composition was prepared:

| | **% by weight** |
|---|---|
| Ammonium persulfate | 20.0 |
| Potassium persulfate | 25.0 |
| Ammonium chloride | 5.0 |
| 2-amino-2-methylpropanol | 3.0 |
| Sodium bicarbonate | 1.5 |
| Sodium metasilicate | 5.0 |
| Diatomaceous earth | ad 100.0 |

Human hair streaks (Caucasian, 21 cm, 2g per bundle) were purchased from Fischbach + Miller Haar, Laupheim, Germany. Each streak was impregnated with 2 g of the reducing composition for 4 min at room temperature. Then the streaks were either rinsed-off with water for 30 s or not rinsed-off. Then 2 g of a mixture of oxidative composition and anhydrous composition (10 g powder per 100 g oxidative composition, delivering pH 9.5) were applied onto the streaks and allowed to process for 30 min at room temperature. Upon treatment, the hair streaks were rinsed-off with water and shampooed with a commercial shampoo available under the trade name Goldwell Dualsenses Deep Cleansing Shampoo. The streaks were then blow-dried. Lightening value L was measured with a Datacolor 45G CT instrument purchased from Datacolor Inc., Lawrenceville, NJ, USA.

The following results were obtained:

| **Treatment groups** | **Prior to treatment** | **Bleached without pre-reduction step** | **Bleached with pre-reduction step (no rinsing)** | **Bleached with pre-reduction step (with rinsing)** |
|---|---|---|---|---|
| | **Lightening L** | | | |
| **Oxidative composition + bleaching powder** | 18.8 | 50.3 | 55.7 | 52.1 |

As a result, the bleaching power for the treatment groups including the pre-reduction step was higher in comparison to the conventional bleaching without the pre-reduction step. Furthermore, the bleaching result with or without rinsing after reductive treatment was not strongly affected by the combination of oxidative treatment + bleaching powder. Moreover, hair feel was similar among all treatment groups indicating no higher degree of damage conferred by reductive pre-treatment. In summary, bleaching was boosted by pre-reduction step with organic reducing agent.

### EXAMPLE 2

The same compositions as in example 1 were used. However, this example is directed to decolorization. Goat hair was purchased from a local farmer. The hair was oxidatively colored with commercially available hair color available under the brand name Goldwell Colorance.

The same processes as described in example 1 were performed on the colored goat hair and the degree of decolorization was measured and reported with lightening value L.

The following results were obtained:

| **Treatment groups** | **Prior to treatment** | **Bleached without pre-reduction step** | **Bleached with pre-reduction step (no rinsing)** | **Bleached with pre-reduction step (with rinsing)** |
|---|---|---|---|---|
| | **Lightening L** | | | |
| **Oxidative composition + bleaching powder** | 17.4 | 40.6 | 52.1 | 49.1 |

As a result, the decolorization power for the treatment groups including the pre-reduction step was higher in comparison to the conventional decolorization without the pre-reduction step. Furthermore, the decolorization results with or without rinsing after reductive treatment was not strongly affected by the combination of oxidative treatment + bleaching powder. Moreover, hair feel was similar among all treatment groups indicating no higher degree of damage conferred by reductive pre-treatment. In summary, decolorization was boosted by pre-reduction step organic reducing agent.

### EXAMPLE 3

The following pre-treatment composition for step a) was prepared by conventional formulation techniques:

| | **% by weight** |
|---|---|
| Cetearyl alcohol | 15.0 |
| 2-amino-2-methylpropanol | 1.8 |
| Disodium hydrogenphophate | 0.5 |
| Sodium sulphite | 1.5 |
| Acrylates copolymer | 0.5 |
| Sodium laureth sulfate | 1.0 |
| EDTA | 0.1 |
| Glycerol | 0.5 |
| Ammonia 25% | q.s. ad pH 9.0 |
| Water | ad 100.0 |

The same oxidative composition comprising hydrogen peroxide and anhydrous composition from example 1 were employed for the present example. The mixing ratios were kept as well.

The treatment process was carried out in analogy to example 1 on human hair for bleaching.

The following results were obtained:

| **Treatment groups** | **Prior to treatment** | **Bleached without pre-reduction step** | **Bleached with pre-reduction step (no rinsing)** | **Bleached with pre-reduction step (with rinsing)** |
|---|---|---|---|---|
| | **Lightening L** | | | |
| **Oxidative composition + bleaching powder** | 18.8 | 50.7 | 54.0 | 54.0 |

As a result, the bleaching power for the treatment groups including the pre-reduction step was higher in comparison to the conventional bleaching without the pre-reduction step. Furthermore, the bleaching results with or without rinsing after reductive treatment was not strongly affected by the combination of oxidative treatment + bleaching powder. Moreover, hair feel was similar among all treatment groups indicating no higher degree of damage conferred by reductive pre-treatment. In summary, bleaching was boosted by pre-reduction step with inorganic reducing agent.

### EXAMPLE 4

The reducing composition of example 3 was employed for the present example, but for decolorization purposes. The oxidative compositions comprising hydrogen peroxide as well as the anhydrous composition were the same as in example 1.

The decolorization process was conducted in analogy to example 1. The following results were obtained on oxidatively colored goat hair:

| **Treatment groups** | **Prior to treatment** | **Bleached without pre-reduction step** | **Bleached with pre-reduction step (no rinsing)** | **Bleached with pre-reduction step (with rinsing)** |
|---|---|---|---|---|
| | **Lightening L** | | | |
| **Oxidative composition + bleaching powder** | 17.4 | 36.6 | 40.1 | 39.8 |

As a result, the decolorization power for the treatment groups including the pre-reduction step was higher in comparison to the conventional decolorization without the pre-reduction step. Furthermore, the decolorization result with or without rinsing after reductive treatment was not strongly affected by the combination of oxidative treatment + bleaching powder. Moreover, hair feel was similar among all treatment groups indicating no higher degree of damage conferred by reductive pre-treatment. In summary, decolorization was boosted by pre-reduction step with inorganic reducing agent.

The following examples are within the scope of the present invention:

### EXAMPLE 5

The following pre-treatment composition for step a) was prepared by conventional formulation techniques:

| | **% by weight** |
|---|---|
| Stearyl alcohol | 7.5 |
| Oleyl alcohol | 5 |
| 2-amino-2-methylpropanol | 1.5 |
| Disodium hydrogenphophate | 0.5 |
| Sodium dithionite | 1.5 |
| Acrylates copolymer | 0.5 |
| Sodium laureth sulfate | 1.0 |
| EDTA | 0.1 |
| Glycerol | 0.5 |
| Ammonia 25% | q.s. ad pH 9.0 |
| Water | ad 100.0 |

The process was followed while employing the oxidative composition comprising hydrogen peroxide and anhydrous composition of example 1.

### EXAMPLE 6

The following pre-treatment composition for step a) was prepared by conventional formulation techniques:

| | **% by weight** |
|---|---|
| Cetearyl alcohol | 15.0 |
| Monoethanolamine | 1.0 |
| Disodium hydrogenphophate | 0.5 |
| Sodium sulfite | 1.7 |
| Acrylates copolymer | 0.5 |
| Sodium laureth sulfate | 1.0 |
| EDTA | 0.1 |
| Glycerol | 0.5 |
| Citric acid | q.s. ad pH 4 |
| Water | ad 100.0 |

The following oxidative composition comprising hydrogen peroxide for step c) was prepared by conventional formulation techniques:

| | **% by weight** |
|---|---|
| Hydrogen peroxide | 12.0 |
| Stearyl alcohol | 6.0 |
| Ceteareth-20 | 1.5 |
| Sodium laureth sulphate | 1.0 |
| Acrylates/beheneth-25 | |
| methacrylate copolymer | 0.5 |
| Phosphoric acid | q.s. ad pH 3.5 |
| Water | ad 100.0 |

The following anhydrous composition was prepared:

| | **% by weight** |
|---|---|
| Ammonium persulfate | 20.0 |
| Potassium persulfate | 30.0 |
| 2-amino-2-methylpropanol | 5.0 |
| Ammonium bicarbonate | 3.0 |
| Sodium metasilicate | 5.0 |
| HC Blue 18 | 0.2 |
| HC Red 18 | 0.1 |
| Diatomaceous earth | ad 100.0 |

2 g of the anhydrous composition was mixed with 10 g of the composition of step c) prior to application onto keratin fibers. The resulting mixture had a pH of 9.5. The process was followed as described in example 1.

### EXAMPLE 7

The following pre-treatment composition for step a) was prepared by conventional formulation techniques:

| | **% by weight** |
|---|---|
| Cetearyl alcohol | 15.0 |
| Disodium hydrogenphophate | 0.5 |
| Sodium sulphite | 2.0 |
| Acrylates copolymer | 0.5 |
| Sodium laureth sulfate | 1.0 |
| EDTA | 0.1 |
| Glycerol | 0.5 |
| Citric acid | q.s. ad pH 3.5 |
| Water | ad 100.0 |

The same oxidative composition comprising hydrogen peroxide and anhydrous compositions from example 1 were employed for the present example. 2 g of the anhydrous composition was mixed with 10 g of the composition of step c) prior to application onto keratin fibers. The resulting mixture had a pH of 9.5. The process was followed as described in example 1.

## Claims

1. A bleaching and/or decolorization process for keratin fibers, preferably human keratin fibers, more preferably human hair, **characterized in that** it comprises the steps of:
a) applying to keratin fibers an aqueous composition comprising a reducing agent at a total concentration in the range of 0.1% to 2% by weight, calculated to the total of the composition, and leaving it onto hair for a time period in the range of 0.5 min to less than 5 min,
b) optionally rinsing-off the keratin fibers,
c) applying to keratin fibers an aqueous oxidizing composition resulting from mixing two compositions prior to application onto hair wherein one of the compositions is an anhydrous composition and comprises one or more peroxy salt(s) and/or persalt(s) and an alkalizing agent, and the other composition is an aqueous composition comprising an oxidizing agent, preferably hydrogen peroxide, wherein the mixture has a pH in the range of 8 to 12 and is left onto hair for a time period of 1 min to 45 min,
d) rinsing-off the keratin fibers,
e) optionally shampooing the keratin fibers,
f) optionally drying the keratin fibers.

2. The process according to claim 1 **characterized in that** the time period for step a) is in the range of 1 min to 4 min.

3. The process according to claims 1 and/or 2 **characterized in that** the reducing agent is selected from organic and/or inorganic reducing agents and/or their mixtures.

4. The process according to any of the preceding claims **characterized in that** the organic reducing agents are selected from thioglycolic acid and/or its salts, thiolactic acid and/or its salts, cysteamine and/or its salts, cysteine and/or its salts, thiourea and/or its salts.

5. The process according to any of the preceding claims **characterized in that** the inorganic reducing agents are selected from sulphite salts, bisulfite salts, dithionate salts.

6. The process according to any of the preceding claims **characterized in that** the pH of the reducing composition is in the range of 2 to 11, preferably it is in the range of 7 to 10.5, more preferably in the range of 8 to 10.

7. The process according to any of the preceding claims **characterized in that** the total concentration of reducing agents in the composition of step a) is in the range of 0.1% to 1.8% by weight, preferably 0.5% to less than 1.5% by weight, calculated to the total of the composition of step a).

8. The process according to any of the preceding claims **characterized in that** the reducing composition comprises one or more alkalizing agent(s), preferably selected from ammonia and/or its salts and/or aminomethyl propanol and/or a an organic alkyl and/or alkanol amine and/or their salts according to the general structure 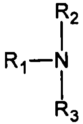 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H.

9. The process according any of the preceding claims **characterized in that** the aqueous reducing composition of step a) and/or the aqueous oxidizing composition of step c) is an emulsion and comprises one or more lipophilic compounds and one or more surfactants.

10. The process according to any of the preceding claims **characterized in that** the reducing composition comprises an organic solvent.

11. The process according to any of the preceding claims **characterized in that** the reducing composition and/or oxidizing composition comprises one or more associative and/or non-associative thickener.

12. The process according to any of the preceding claims **characterized in that** the aqueous oxidizing composition comprises hydrogen peroxide at a concentration in the range of 1% to 20% by weight, more preferably in the range of 2% to 15% by weight, most preferably in the range of 3% to 12% by weight, calculated to the total of the aqueous oxidizing composition.

13. The process according to any of the preceding claims **characterized in that** the anhydrous composition comprises one or more persalts and/or peroxy salts at a concentration in the range of 10 to 80% by weight calculated to the total of the anhydrous composition.

14. The process according to any of the preceding claims **characterized in that** the anhydrous composition comprises one or more ammonium salt(s) other than persalt(s) and peroxy salt(s).

15. Kit-of-parts for bleaching and/or decolorizing keratin fibers, preferably human keratin fibers, more preferably human hair, **characterized in that** it comprises in a first separately packed container a composition as defined in the claims 1 to 14 for step a), in a second separately packed container an anhydrous composition as defined in the claims 1 to 14 for step c), and in a third separately packed container an aqueous composition comprising an oxidizing agent, preferably hydrogen peroxide, as defined in the claims 1 to 14 for step c).

## Patentansprüche

1. Verfahren zum Bleichen und/oder Entfärben von Keratinfasern, vorzugsweise menschlichen Keratinfasern, noch bevorzugtes menschliches Haar, **dadurch gekennzeichnet, dass** es die folgenden Schritte umfasst:
a) Aufbringen einer wässrigen Zusammensetzung, die ein Reduktionsmittel in einer Gesamtkonzentration im Bereich von 0.1 bis 2 Gew.-% enthält, berechnet auf die Gesamtzusammensetzung, auf die Keratinfasern und Belassen dieser Zusammensetzung auf dem Haar für einen Zeitraum im Bereich von 0.5 min bis weniger als 5 min,
b) gegebenenfalls Abspülen der Keratinfasern,
c) Aufbringen einer wässrigen oxidierenden Zusammensetzung auf die Keratinfasern, die durch Mischen zweier Zusammensetzungen vor dem Aufbringen auf das Haar entsteht, wobei eine der Zusammensetzungen eine wasserfreie Zusammensetzung ist und ein oder mehrere Peroxysalze und/oder Persalze und ein Alkalisierungsmittel umfasst, und die andere Zusammensetzung eine wässrige Zusammensetzung ist, die ein Oxidationsmittel, vorzugsweise Wasserstoffperoxid, umfasst, wobei die Mischung einen pH-Wert im Bereich von 8 bis 12 aufweist und für einen Zeitraum von 1 Minute bis 45 Minuten auf dem Haar belassen wird,
d) Ausspülen der Keratinfasern,
e) optionales Waschen der Keratinfasern mit Shampoo,
f) gegebenenfalls Trocknen der Keratinfasern.

2. Verfahren nach Anspruch 1, **dadurch gekennzeichnet, dass** die Zeitdauer für Schritt a) im Bereich von 1 Minute bis 4 Minuten liegt.

3. Verfahren nach Anspruch 1 und/oder 2, **dadurch gekennzeichnet, dass** das Reduktionsmittel aus organischen und/oder anorganischen Reduktionsmitteln und/oder deren Mischungen ausgewählt ist.

4. Verfahren nach einem der vorstehenden Ansprüche, **dadurch gekennzeichnet, dass** die organischen Reduktionsmittel ausgewählt sind aus Thioglykolsäure und/oder deren Salzen, Thiolactinsäure und/oder deren Salzen, Cysteamin und/oder dessen Salzen, Cystein und/oder dessen Salzen, Thioharnstoff und/oder dessen Salzen.

5. Verfahren nach einem der vorstehenden Ansprüche, **dadurch gekennzeichnet, dass** die anorganischen Reduktionsmittel aus Sulfitsalzen, Bisulfitsalzen und Dithionatsalzen ausgewählt sind.

6. Verfahren nach einem der vorstehenden Ansprüche, **dadurch gekennzeichnet, dass** der pH-Wert der reduzierenden Zusammensetzung im Bereich von 2 bis 11 liegt, vorzugsweise im Bereich von 7 bis 10.5, noch bevorzugter im Bereich von 8 bis 10.

7. Verfahren nach einem der vorstehenden Ansprüche, **dadurch gekennzeichnet, dass** die Gesamtkonzentration der Reduktionsmittel in der Zusammensetzung von Schritt a) im Bereich von 0.1 bis 1.8 Gew.-%, vorzugsweise 0.5 bis weniger als 1.5 Gew.-%, bezogen auf die Gesamtmenge der Zusammensetzung von Schritt a), liegt.

8. Verfahren nach einem der vorstehenden Ansprüche, **dadurch gekennzeichnet, dass** die reduzierende Zusammensetzung ein oder mehrere Alkalisierungsmittel umfasst, vorzugsweise ausgewählt aus Ammoniak und/oder seinen Salzen und/oder Aminomethylpropanol und/oder einem organischen Alkyl- und/oder Alkanolamin und/oder deren Salzen gemäß der allgemeinen Struktur 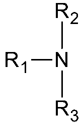 wobei R1, R2 und R3 unabhängig voneinander ausgewählt sind aus H, linearem C1-C6-Alkyl, das mit einer Hydroxylgruppe substituiert sein kann, oder verzweigtem C3-C12-Alkyl oder Alkanol, wobei mindestens eines von R1, R2 oder R3 von H verschieden ist.

9. Verfahren nach einem der vorstehenden Ansprüche, **dadurch gekennzeichnet, dass** die wässrige reduzierende Zusammensetzung von Schritt a) und/oder die wässrige oxidierende Zusammensetzung von Schritt c) eine Emulsion ist und eine oder mehrere lipophile Verbindungen und ein oder mehrere Tenside umfasst.

10. Verfahren nach einem der vorstehenden Ansprüche, **dadurch gekennzeichnet, dass** die reduzierende Zusammensetzung ein organisches Lösungsmittel umfasst.

11. Verfahren nach einem der vorstehenden Ansprüche, **dadurch gekennzeichnet, dass** die reduzierende Zusammensetzung und/oder die oxidierende Zusammensetzung ein oder mehrere assoziative und/oder nicht-assoziative Verdickungsmittel umfasst.

12. Verfahren nach einem der vorstehenden Ansprüche, **dadurch gekennzeichnet, dass** die wässrige Oxidationszusammensetzung Wasserstoffperoxid in einer Konzentration im Bereich von 1 bis 20 Gew.-%, vorzugsweise im Bereich von 2 bis 15 Gew.-%, am bevorzugtesten im Bereich von 3 bis 12 Gew.-%, berechnet auf die Gesamtmenge der wässrigen Oxidationszusammensetzung, umfasst.

13. Verfahren nach einem der vorstehenden Ansprüche, **dadurch gekennzeichnet, dass** die wasserfreie Zusammensetzung ein oder mehrere Persalze und/oder Peroxysalze in einer Konzentration im Bereich von 10 bis 80 Gew.-%, berechnet auf die Gesamtmenge der wasserfreien Zusammensetzung, umfasst.

14. Verfahren nach einem der vorstehenden Ansprüche, **dadurch gekennzeichnet, dass** die wasserfreie Zusammensetzung ein oder mehrere Ammoniumsalze außer Persalzen und Peroxysalzen umfasst.

15. Kit zum Bleichen und/oder Entfärben von Keratinfasern, vorzugsweise menschlichen Keratinfasern, noch bevorzugter menschlichem Haar, **dadurch gekennzeichnet, dass** er in einem ersten separat verpackten Behälter eine Zusammensetzung gemäß den Ansprüchen 1 bis 14 für Schritt a) enthält, in einem zweiten separat verpackten Behälter eine wasserfreie Zusammensetzung gemäß den Ansprüchen 1 bis 14 für Schritt c) enthält und in einem dritten separat verpackten Behälter eine wässrige Zusammensetzung, die ein Oxidationsmittel, vorzugsweise Wasserstoffperoxid, gemäß den Ansprüchen 1 bis 14 für Schritt c) umfasst.

## Revendications

1. Procédé de blanchiment et/ou de décoloration pour fibres kératiniques, de préférence des fibres kératiniques humaines, plus préférentiellement des cheveux humains, **caractérisé en ce qu'**il comprend les étapes consistant à :
a) l'application sur les fibres kératiniques d'une composition aqueuse comprenant un agent réducteur à une concentration totale comprise entre 0.1 % et 2 % en poids, calculée par rapport au total de la composition, et son maintien sur les cheveux pendant une durée comprise entre 0.5 minute et moins de 5 minutes,
b) rincer éventuellement les fibres kératiniques,
c) appliquer sur les fibres kératiniques une composition oxydante aqueuse résultant du mélange de deux compositions avant l'application sur les cheveux, dans laquelle l'une des compositions est une composition anhydre et comprend un ou plusieurs sels peroxy et/ou persels et un agent alcalinisant, et l'autre composition est une composition aqueuse comprenant un agent oxydant, de préférence du peroxyde d'hydrogène, dans laquelle le mélange a un pH compris entre 8 et 12 et est laissé sur les cheveux pendant une durée comprise entre 1 minute et 45 minutes,
d) rinçage des fibres kératiniques,
e) éventuellement, laver les fibres kératiniques avec un shampooing,
f) sécher éventuellement les fibres kératiniques.

2. Procédé selon la revendication 1, **caractérisé en ce que** la durée de l'étape a) est comprise entre 1 minute et 4 minutes.

3. Procédé selon les revendications 1 et/ou 2, **caractérisé en ce que** l'agent réducteur est choisi parmi les agents réducteurs organiques et/ou inorganiques et/ou leurs mélanges.

4. Procédé selon l'une quelconque des revendications précédentes, **caractérisé en ce que** les agents réducteurs organiques sont choisis parmi l'acide thioglycolique et/ou ses sels, l'acide thiolactique et/ou ses sels, la cystéamine et/ou ses sels, la cystéine et/ou ses sels, la thiourée et/ou ses sels.

5. Procédé selon l'une quelconque des revendications précédentes, **caractérisé en ce que** les agents réducteurs inorganiques sont choisis parmi les sels de sulfite, les sels de bisulfite, les sels de dithionate.

6. Procédé selon l'une quelconque des revendications précédentes, **caractérisé en ce que** le pH de la composition réductrice est compris entre 2 et 11, de préférence entre 7 et 10.5, et plus préférablement entre 8 et 10.

7. Procédé selon l'une quelconque des revendications précédentes, **caractérisé en ce que** la concentration totale en agents réducteurs dans la composition de l'étape a) est comprise entre 0.1 % et 1.8 % en poids, de préférence entre 0.5 % et moins de 1.5 % en poids, calculée par rapport au total de la composition de l'étape a).

8. Procédé selon l'une quelconque des revendications précédentes, **caractérisé en ce que** la composition réductrice comprend un ou plusieurs agents alcalinisants, de préférence choisis parmi l'ammoniac et/ou ses sels et/ou l'aminométhylpropanol et/ou une alkylamine et/ou une alcanolamine organique et/ou leurs sels selon la structure générale 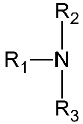 dans laquelle R1, R2 et R3 sont choisis indépendamment parmi H, un groupe alkyle linéaire en C1-C6 qui peut être substitué par un groupe hydroxyle, ou un groupe alkyle ou alcanol ramifié en C3-C12, dans lequel au moins l'un des groupes R1, R2 ou R3 est différent de H.

9. Procédé selon l'une quelconque des revendications précédentes, **caractérisé en ce que** la composition réductrice aqueuse de l'étape a) et/ou la composition oxydante aqueuse de l'étape c) est une émulsion et comprend un ou plusieurs composés lipophiles et un ou plusieurs tensioactifs.

10. Procédé selon l'une quelconque des revendications précédentes, **caractérisé en ce que** la composition réductrice comprend un solvant organique.

11. Procédé selon l'une quelconque des revendications précédentes, **caractérisé en ce que** la composition réductrice et/ou la composition oxydante comprend un ou plusieurs épaississants associatifs et/ou non associatifs.

12. Procédé selon l'une quelconque des revendications précédentes, **caractérisé en ce que** la composition oxydante aqueuse comprend du peroxyde d'hydrogène à une concentration comprise dans la plage de 1 % à 20 % en poids, de préférence dans la plage de 2 % à 15 % en poids, de préférence dans la plage de 3 % à 12 % en poids, calculée par rapport au total de la composition oxydante aqueuse.

13. Procédé selon l'une quelconque des revendications précédentes, **caractérisé en ce que** la composition anhydre comprend un ou plusieurs persels et/ou peroxy-sels à une concentration comprise entre 10 et 80 % en poids, calculée par rapport au total de la composition anhydre.

14. Procédé selon l'une quelconque des revendications précédentes, **caractérisé en ce que** la composition anhydre comprend un ou plusieurs sels d'ammonium autres que des persels et des sels peroxy.

15. Kit de pièces pour blanchir et/ou décolorer des fibres kératiniques, de préférence des fibres kératiniques humaines, plus préférablement des cheveux humains, **caractérisé en ce qu'**il comprend dans un premier récipient emballé séparément une composition telle que définie dans les revendications 1 à 14 pour l'étape a), dans un deuxième récipient emballé séparément une composition anhydre telle que définie dans les revendications 1 à 14 pour l'étape c), et dans un troisième récipient emballé séparément, une composition aqueuse comprenant un agent oxydant, de préférence du peroxyde d'hydrogène, tel que défini dans les revendications 1 à 14 pour l'étape c).
